(19) Europäisches Patentamt
European Patent Office
Office européen des brevets

(11) Veröffentlichungsnummer: **0 175 241**
**B1**

# (12) EUROPÄISCHE PATENTSCHRIFT

(45) Veröffentlichungstag der Patentschrift: **03.08.88**

(21) Anmeldenummer: **85111315.9**

(22) Anmeldetag: **07.09.85**

(51) Int. Cl.⁴: **C 07 D 317/34**, C 07 D 317/72

**ERRATUM**

| DIE TEXTSTELLE: TEXT PUBLISHED: LE PASSAGE SUIVANT: | | (SEITE, SPALTE, ZEILE) (PAGE, COLUMN, LINE) (PAGE, COLONNE, LIGNE) | | LAUTET BERICHTIGT: SHOULD READ: DEVRAIT ETRE LU: | |
|---|---|---|---|---|---|
| 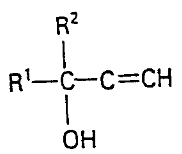 | II | 2 | 1 | 23/29 | 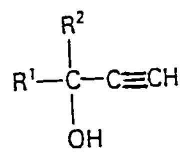 II |
| Quaternierung mit einem $C_6$-$C_6$- | | 2 | 2 | 13 | Quaternierung mit einem $C_1$-$C_6$- |
| Methyl-1-in-3-ols, etwa | | 2 | 2 | 39 | but-1-in-3 Methyl-ols, etwa |
| Auch die Reaktionstemperatur, | | 2 | 2 | 55 | Auch die Reaktionstemperaturen, |
| DE-C-1 098 953 in Bereich | | 2 | 2 | 56 | DE-C-1 098 963 ein Bereich |
| Als tertiäre Base kann | | 3 | 3 | 12 | Als tertiäre Basen kann |
| 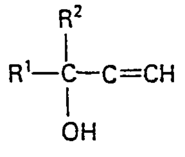 | II | 4<br>5<br>5 | 6<br>7<br>8 | 46/52<br>30/36<br>23/29 | 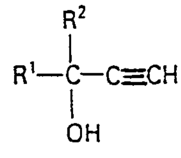 II |

Tag der Entscheidung über die Berichtigung / Date of decision on rectification / Date de décision portant sur modification: 07.11.88

Ausgabe- und Veröffentlichungstag / Issue and publication date / Date d'edition et de publication: 11.01.89

Patbl.Nr / EPB no / Bull. no: 89/02

(19) Europäisches Patentamt
European Patent Office
Office européen des brevets

(11) Veröffentlichungsnummer: **0 175 241**
**B1**

# (12) EUROPÄISCHE PATENTSCHRIFT

(45) Veröffentlichungstag der Patentschrift: **03.08.88**

(51) Int. Cl.⁴: **C 07 D 317/34**, C 07 D 317/72

(21) Anmeldenummer: **85111315.9**

(22) Anmeldetag: **07.09.85**

(54) **Verfahren zur Herstellung von 4,4-disubstituierten 5-Methylen-1,3-dioxolan-2-onen.**

(30) Priorität: **12.09.84 DE 3433403**

(43) Veröffentlichungstag der Anmeldung: **26.03.86 Patentblatt 86/13**

(45) Bekanntmachung des Hinweises auf die Patenterteilung: **03.08.88 Patentblatt 88/31**

(84) Benannte Vertragsstaaten: **BE CH DE FR GB IT LI NL**

(56) Entgegenhaltungen:
**EP-A-0 067 361**
**DE-B-1 098 953**
**US-A-4 231 937**

(73) Patentinhaber: **BASF Aktiengesellschaft, Carl-Bosch-Strasse 38, D-6700 Ludwigshafen (DE)**

(72) Erfinder: **Schneider, Kurt, Dr., Auf dem Koeppel, D-6702 Bad Duerkheim (DE)**
Erfinder: **Best, Walter, Dr., Im Spiess 2, D-6714 Weisenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 175 241 B1

ACTORUM AG

**Beschreibung**

Die vorliegende Erfindung betrifft ein verbessertes Verfahren zur Herstellung von 4,4-disubstituierten 5-Methylen-1,3-dioxolan-2-onen der allgemeinen Formel I

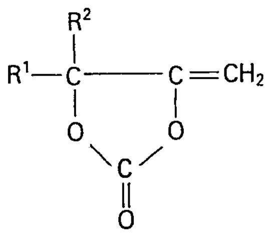

I

in der $R^1$ für einen unter den Reaktionsbedingungen inerten, organischen Rest und $R^2$ für eine $C_1$–$C_4$-Alkylgruppe stehen, wobei $R^1$ und $R^2$ auch zu einem 5- oder 6-gliedrigen Ring verbunden sein können, durch Umsetzung von Prop-1-in-3-olen der allgemeinen Formel II

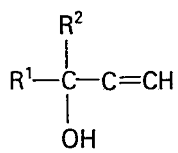

II

mit Kohlendioxid in Gegenwart von Kupfersalzen und tertiären Basen als Katalysatoren.

Dieses Verfahren ist, sieht man von der erfindungsgemässen Verbesserung ab, aus der DE-C-1098953 bekannt, vermag aber sofern nicht zu befriedigen, als es entweder zu geringe Raum-Zeit-Ausbeuten liefert oder unwirtschaftlich scharfe Reaktionsbedingungen erfordert.

Entsprechend der Aufgabe, diesen Nachteilen abzuhelfen, wurde eine Verbesserung des eingangs definierten Verfahrens gefunden, welche dadurch gekennzeichnet ist, dass man es in Gegenwart wirksamer Mengen eines quartären Ammonium- oder Phosphoniumsalzes III vornimmt.

Als quartäre Salze III eignen sich prinzipiell alle Ammonium- und Phosphoniumsalze, darunter solche der allgemeinen Formel IIIa und IIIb

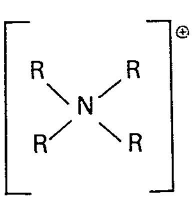 $X^{\ominus}$ und 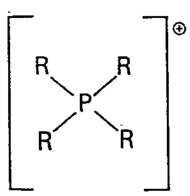 $X^{\ominus}$

IIIa IIIb

in denen die Substituenten R gleiche oder verschiedene Kohlenwasserstoffreste mit je 1–20 C-Atomen bedeuten, wobei die Summe der C-Atome in den Resten R jeweils nicht grösser als 24 ist, und in denen X Halogen, vorzugsweise Brom, bedeutet.

Wegen der generellen Eignung der Salze III richtet sich deren Wahl hauptsächlich nach deren Verfügbarkeit und nach deren Preis. Praktisch wird man daher vor allem mit den Ammoniumsalzen IIIa arbeiten, wobei das handelsübliche und leicht herzustellende Tetraethylammoniumbromid an erster Stelle zu nennen ist. Daneben sind diejenigen Verbindungen IIIa hervorzuheben, in denen drei der Reste R niedere Alkylgruppen wie die Methyl- oder Ethylgruppe und der vierte den Benzylrest oder einen unverzweigten $C_6$-$C_{18}$-Alkylrest bedeuten.

Unter den Phosphoniumsalzen IIIb sind diejenigen am besten zugänglich, die sich vom Triphenylphosphin ableiten und deren vierter Substituent durch Quaternierung mit einem $C_6$–$C_6$-Alkylbromid in das Molekül eingeführt wurde.

Allgemein können die Kohlenwasserstoffreste in den Verbindungen IIIa und IIIb verzweigte oder vorzugsweise unverzweigte $C_1$–$C_{20}$-Alkylgruppen, Aralkylgruppen wie die Benzylgruppe, die Cyclohexylgruppe und aromatische Gruppen wie die Phenyl- oder die p-Tolylgruppe bedeuten. Ferner können Alkylreste R auch miteinander verbunden sein, etwa unter Ausbildung eines Piperidinringes oder eines entsprechenden phosphorheterocyclischen Ringes.

Häufig, und zwar besonders im Falle der Bromide, ist es nicht erforderlich, von den Salzen III selber auszugehen, sondern es genügt, deren Vorprodukte Base und Quaternierungsreagens einzusetzen, aus denen sich die wirksamen Quaternierungsprodukte III von selber bilden.

Die Menge der Oniumsalze III ist im Prinzip beliebig, da sie nach den bisherigen Beobachtungen lediglich einen Einfluss auf die Reaktionsgeschwindigkeit hat, deren Grössenordnung sich ihrerseits nach der Art des eingesetzten Propinols II richtet. Für die technisch befriedigenden Reaktionszeiten von etwa 0,5–5 h benötigt man im Falle reaktiver Propinole II, beispielsweise des 3-Methyl-1-in-3-ols, etwa 1–5 mmol III/mol II, und für den Fall weniger reaktionsfreudiger Propinole II verwendet man vorzugsweise 5–10 mmol III/mol II.

Es ist ein besonderer Vorgang des erfindungsgemässen Verfahrens, dass es bereits das erfolgreiche Arbeiten bei Normaldruck gestattet und dass ein höherer Druck als 20 bar in der Regel nicht erforderlich ist. Andererseits kann man die Umsetzung ohne weiteres auch unter einem beliebig hohen Druck ausführen, etwa wenn es sich um ein sehr reaktionsträges Propinol II handelt oder wenn man auf besonders kurze Reaktionszeiten Wert legt. Das Arbeiten über 100 bar dürfte aber nur in Ausnahmefällen noch einen merklichen Vorteil mit sich bringen.

Auch die Reaktionstemperatur, für die sich nach dem Verfahren der DE-C-1098953 in Bereich von bis zu 200 °C empfiehlt, lassen sich nach dem erfindungsgemässen Verfahren erheblich erniedrigen, und zwar auf einen Bereich von 50–100 °C. Höhere Temperaturen, etwa bis 200 °C sind daher in der Regel nur im Falle reaktionsträger Propinole II anzuwenden.

Wie beim grundlegenden Verfahren der DE-C-1098953 dienen Kupfersalze und tertiäre Basen als weitere katalytisch aktive Reaktionspartner.

Als Kupfersalze eignen sich prinzipiell alle ionischen Verbindungen des ein- und zweiwertigen Kupfers, z.B. die Oxide, Hydroxide, Sulfate, Nitrate und Phosphate, besonders aber die Halogenide und die Salze von $C_2$–$C_{16}$-Fettsäuren, wie vor allem die Acetate.

Die Menge der Kupfersalze ist grundsätzlich beliebig, jedoch verwendet man sie zur Erzielung befriedigender Reaktionsgeschwindigkeiten, vorzugsweise in Mengen von 1–10 mmol/mol II.

Als tertiäre Base kann man Trialkylamine wie Triethylamin und heterocyclische Stickstoffbasen wie Pyridin verwenden. Bevorzugt werden tertiäre Phosphine wie Tributylphosphin und Triphenylphosphin verwendet. Die Menge der Base ist nicht kritisch und liegt im allgemeinen zwischen 1 und 10 mmol/mol II.

Die Umsetzung wird vorzugsweise ohne Lösungsmittel ausgeführt, jedoch kann ein inertes Lösungsmittel wie Dioxan, Toluol, Aceton oder Glycoldiethylether in Mengen von etwa 25–200 Gew.-%, bezogen auf das eingesetzte Propinol II mitverwendet werden. Ist das Verfahrensprodukt I unter den Reaktionsbedingungen flüssig, verwendet man zweckmässigerweise dieses als Lösungsmittel. Damit die Verfahrensprodukte nicht hydrolysieren, empfiehlt es sich, unter Ausschluss von Wasser zu arbeiten.

Verfahrenstechnisch bietet das erfindungsgemässe Verfahren keinerlei Besonderheiten, d.h. man kann es wie üblich diskontinuierlich oder kontinuierlich vornehmen, indem man eine Mischung aus II, den Katalysatorbestandteilen und gegebenenfalls dem Lösungsmittel unter intensiver Durchmischung unter den Reaktionsbedingungen mit dem Kohlendioxid in Kontakt bringt.

Normalerweise erzielt man praktisch quantitative Umsätze an II, wonach man das Reaktionsgemisch wie üblich aufarbeitet. Hierbei anfallende katalysatorhaltige Rückstände können meist mehrmals für weitere Reaktionssätze verwendet werden.

Nach den bisherigen Beobachtungen ist das gute Gelingen des erfindungsgemässen Verfahrens von der Art des eingesetzten Propinols II nicht abhängig, sofern der Rest $R^1$, wie sich allerdings von selbst versteht, keine unter den Reaktionsbedingungen nicht-inerten Substituenten enthält.

Als Reste $R^1$ seien genannt:

- Gesättigte und ungesättigte verzweigte und unverzweigte aliphatische Reste mit bis zu 40 C-Atomen,
- iso- oder heterocyclische cycloaliphatische Gruppen mit vorzugsweise 5–7 Ringgliedern,
- iso- oder heterocyclische aromatische Gruppen,
- gemischte Reste mit Gruppierungen der vorgenannten Art, beispielsweise araliphatische Reste wie die Benzylgruppe, und
- mit $R^2$ zu 5- oder 6-Ringen verbundene Reste.

Diese Gruppen können Substituenten wie Halogen, die Nitrogruppe, freie oder substituierte Aminogruppen, die Hydroxylgruppe, die Formylgruppe oder die Cyangruppe tragen oder Ether-, Keton- oder Estergruppierungen enthalten.

Bei den Resten $R^2$ handelt es sich in der Regel um die Methylgruppe und auch um mit $R^1$ verbundene Reste, beispielsweise um den Cyclohexylidenrest.

Beispiele für Propinole II sind 3-Methylbut-1-in-3-ol, 3-Methylpent-1-in-3-ol, 3-Ethylpent-1-in-3-ol, 1-Ethinylcyclohexan-1-ol und Dehydrolinalool (3,7-Dimethyloct-6-en-1-in-3-ol).

Die Verfahrensprodukte I sind wertvolle Zwischenprodukte für organische Synthesen.

Beispiel 1

Herstellung von 4,4-Dimethyl-5-methylen-1,3-dioxolan-2-on

Je 1260 g (15 mol) 3-Methylbut-1-in-3-ol wurden bei 80 °C und einem $CO_2$-Druck von 20 bar mit einem Kupfersalz, einer tertiären Base und Tetraethylammoniumbromid (TEAB) sowie im Vergleich dazu ohne TEAB umgesetzt.

Einzelheiten über Art und Menge der Katalysatorbestandteile, über die Reaktionsdauer und über die Ausbeuten an dem Dioxolanon sind der nachstehenden Tabelle zu entnehmen.

Tabelle

| Versuch | Cu-Salz [g], [mol.-%]* | tert. Base [g], [mol.-%]* | TEAB [g], [mol.-%]* | Dauer [h] | Ausbeute an Dioxolanon [%] |
|---|---|---|---|---|---|
| erfindungsgemäss | | | | | |
| a | Cu-I-Iodid | $PPh_3$ | | 12 | 96 |
| | 10; 0,35 | 50; 1,27 | 10; 0,32 | | |
| b | Cu-II-Acetat | $Net_3$ | | 12 | 98 |
| | 5; 0,18 | 10; 0,66 | 10; 0,32 | | |
| c | Cu-II-Acetat | $PPh_3$ | | | |
| | 5; 0,18 | 15; 0,39 | 5; 0,16 | 3,5 | 99 |
| d | Cu-II-Acetat | $Ph_3$ | | 2,5 | 99 |
| | 5; 0,18 | 10; 0,26 | 10; 0,16 | | |
| e | Cu-II-Acetat | $PPh_3$ | | 2 | 99 |
| | 5; 0,18 | 5; 0,13 | 15; 0,48 | | |

* bezogen auf Methylbutinol

Tabelle

| Versuch | Cu-Salz [g], [mol.-%]* | tert. Base [g], [mol.-%]* | TEAB [g], [mol.-%]* | Dauer [h] | Ausbeute an Dioxolanon [%] |
|---|---|---|---|---|---|
| zum Vergleich | | | | | |
| f | Cu-I-Iodid 10; 0,35 | $Net_3$ 30; 1,98 | – | 36 | 67 |
| g | Cu-II-Acetat 5; 0,18 | $Net_3$ 20; 1,32 | – | 18 | 3 |

* bezogen auf Methylbutinol

Beispiel 2
Herstellung von 4-Methyl-4-(4-methylpent-1-yl)-5-methylen-dioxolan-2-on

Diese Verbindung wurde analog Beispiel 1 aus 924 g (6 mol) 3,7-Dimethyloct-1-in-3-ol, 5,53 g (0,5 mol.-%, bezogen auf das Octinol) Cu-II-Acetat, 7,93 g (0,5 mol.-%, bezogen auf das Octinol) Triphenylphosphin und 6,29 g (0,5 mol.-%, bezogen auf das Octinol) TEAB in 98%iger Ausbeute sowie in einer Reinheit von 98% hergestellt, wobei die Reaktionsdauer 8 h betrug.

Beispiel 3
Herstellung von 4-Methyl-4-(4-methylpent-3-en-1-yl)-5-methylen-dioxolan-2-on

Diese Verbindung wurde auf die in Beispiel 2 angegebene Weise aus 912 g (6 mol) 3,7-Dimethyloct-6-en-1-in-3-ol (Dehydrolinalool) in 98%iger Ausbeute und 98%iger Reinheit hergestellt; die Reaktionsdauer betrug 9 h.

Beispiel 4
Herstellung von 4,4-Pentamethylen-5-methylen-dioxolan-2-on

1240 g (10 mol) Ethinylcyclohexanol, 5 g (0,28 mol.-%, bezogen auf das Alkinol) Cu-II-Acetat, 10 g (0,38 mol.-%, bezogen auf das Alkinol) Triphenylphosphin und 10 g (0,48 mol.-%, bezogen auf das Alkinol) TEAB wurden im Laufe von 7,5 h auf die in Beispiel 1 angegebene Weise zu der oben genannten Verbindung umgesetzt. Ausbeute und Reinheit betrugen je 98%.

Beispiel 5
Herstellung von 4-Methyl-4-ethyl-5-methylen-dioxolan-2-on

Diese Verbindung wurde auf die in Beispiel 4 angegebene Weise aus 1176 g (12 mol) 3-Methylpent-1-in-3-ol in 98%iger Ausbeute und einer Reinheit von 99% hergestellt; die Reaktionszeit betrug 5 h.

Beispiel 6
Herstellung von 4,4-Dimethyl-5-methylen-dioxolan-2-on

Analog Beispiel 1e, jedoch mit 20 g (0,41 mol.-%, bezogen auf das eingesetzte Butinol) Tetrabutylammoniumbromid anstelle des TEAB, fiel das Dixolanon in einer Ausbeute und einer Reinheit von 99% an; die Reaktionsdauer betrug 2,5 h.

Beispiel 7
Herstellung von 4,4-Dimethyl-5-methylen-dioxolan-2-on

Analog Beispiel 1e, jedoch mit 25 g (0,45 mol.-%, bezogen auf das Butinol) Ethyltriphenylphosphoniumbromid anstelle des TEAB, fiel das Dioxolanon in einer Ausbeute und einer Reinheit von je 99% an; die Reaktionsdauer betrug 3 h.

**Patentansprüche**

1. Verfahren zur Herstellung von 4,4-disubstituierten 5-Methylen-1,3-dioxolan-2-onen der allgemeinen Formel I

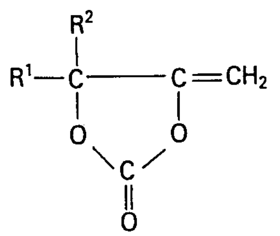

I

in der $R^1$ für einen unter den Reaktionsbedingungen inerten, organischen Rest und $R^2$ für eine $C_1$–$C_4$-Alkylgruppe stehen, wobei $R^1$ und $R^2$ auch zu einem 5- oder 6-gliedrigen Ring verbunden sein können, durch Umsetzung von Prop-1-in-3-olen der allgemeinen Formel II

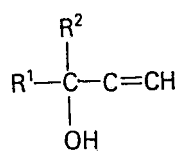

II

mit Kohlendioxid in Gegenwart von Kupfersalzen und tertiären Basen als Katalysatoren, dadurch gekennzeichnet, dass man es in Gegenwart wirksamer Mengen eines quartären Ammonium- oder Phosphoniumsalzes III vornimmt.

2. Verfahren nach Anspruch 1, dadurch gekennzeichnet, dass man es bei 1–20 bar vornimmt.

3. Verfahren nach den Ansprüchen 1 und 2, dadurch gekennzeichnet, dass man als quartäres Ammoniumsalz Tetraethylammoniumbromid verwendet.

4. Verfahren nach den Ansprüchen 1 bis 3, dadurch gekennzeichnet, dass man als Kupfersalz Kupfer-II-Acetat verwendet.

5. Verfahren nach den Ansprüchen 1 bis 4, dadurch gekennzeichnet, dass man als tertiäre Base Triphenylphosphin verwendet.

**Claims**

1. A process for the preparation of a 4,4-disubstituted 5-methylene-1,3-dioxolan-2-one of the general formula I

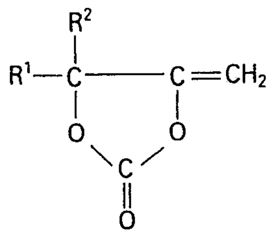

I

where $R^1$ is an organic radical which is inert under the reaction conditions, and $R^2$ is $C_1$–$C_4$-alkyl, and $R^1$ and $R^2$ may furthermore be bonded to form a 5-membered or 6-membered ring, by reacting a prop-1-yn-3-ol of the general formula II

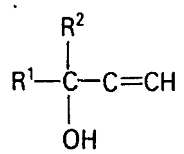

II

with carbon dioxide in the presence of a copper salt and a tertiary base as catalysts, which process is carried out in the presence of an effective amount of a quaternary ammonium or phosphonium salt III.

2. A process as claimed in claim 1, which is carried out under 1–20 bar.

3. A process as claimed in claims 1 and 2, wherein the quaternary ammonium salt used is tetraethylammonium bromide.

4. A process as claimed in claims 1 to 3, wherein the copper salt used is copper(II) acetate.

5. A process as claimed in claims 1 to 4, wherein the tertiary base used is triphenylphosphine.

**Revendications**

1. Procédé de préparation de 5-méthylène-1,3-dioxolane-2-ones 4,4-disubstituées de formule générale I

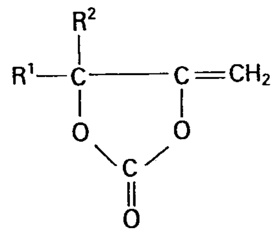

I

dans laquelle $R^1$ est mis pour un radical organique inerte dans les conditions de la réaction et $R^2$ pour un groupe alkyle en $C_1$–$C_4$, $R^1$ et $R^2$ pouvant être également unis en un noyau pentagonal ou hexagonal, par réaction de prop-1-yne-3-ols de formule générale II

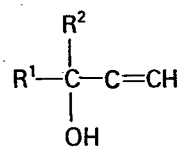

II

avec l'anhydride carbonique en présence de sels de cuivre et de bases tertiaires servant de catalyseurs, caractérisé en ce qu'on procède en présence de quantités actives d'un sel d'ammonium ou de phosphonium quaternaire III.

2. Procédé selon la revendication 1, caractérisé en ce qu'on procède sous 1 à 20 bars.

3. Procédé selon la revendication 1 ou 2, caractérisé en ce qu'on utilise, en tant que sel d'ammonium quaternaire, le bromure de tétraéthylammonium.

4. Procédé selon l'une quelconque des revendications 1 à 3, caractérisé en ce qu'on utilise, en tant que sel de cuivre, l'acétate cuivrique.

5. Procédé selon l'une quelconque des revendications 1 à 4, caractérisé en ce qu'on utilise, en tant que base tertiaire, la triphénylphosphine.